# EUROPÄISCHE PATENTSCHRIFT

(11) **EP 4 231 974 B1**
(45) Veröffentlichungstag und Bekanntmachung des Hinweises auf die Patenterteilung: **24.07.2024**
(21) Anmeldenummer: 21798951.6
(22) Anmeldetag: 13.10.2021
(51) Int. Cl.: A61F 5/01, A61F 2/66, A61F 2/76, A61F 2/60

(54) **EXTREMITÄTENDUMMY UND VERFAHREN ZUM TESTEN DER FUNKTION EINER ORTHESE**
EXTREMITY DUMMY AND METHOD FOR TESTING THE FUNCTION OF AN ORTHOSIS
TEST DE L'EXTREMITE ET PROCEDURE POUR TESTER LE FONCTIONNEMENT D'UNE ORTHHESE

(30) Priorität: 23.10.2020 DE 102020127967
(43) Veröffentlichungstag der Anmeldung: 30.08.2023
(73) Patentinhaber: Universität Paderborn, 33098 Paderborn (DE)
(72) Erfinder: KULLMER, Gunter, 33184 Altenbeken (DE); SCHRAMM, Britta, 33102 Paderborn (DE); SCHAFRAN, Tommy, 59427 Unna (DE); SCHRAEDER, Dirk-Theodor, 32756 Detmold (DE)
(74) Vertreter: Cohausz Hannig Borkowski Wißgott
(86) Internationale Anmeldenummer: PCT/EP2021/078252
(87) Internationale Veröffentlichungsnummer: WO 2022/084112

(56) Entgegenhaltungen:
- WO-A1-2019/045646
- AMANDA WACH: "Mechanical Characterization of Carbon Fiber and Thermoplastic Ankle Foot Orthoses", MASTER'S THESES DISSERTATION, vol. 341, 1 December 2015 (2015-12-01), XP055581088
- D.J.J BREGMAN: "The Optimal Ankle Foot Orthosis: The influence of mechanical properties of Ankle Foot Orthoses on the walking ability of patients with central neurological disorders", VRIJE UNIVERSITEIT AMSTERDAM PHD THESIS DISSERTATION, 1 January 2011 (2011-01-01), XP055581053

## Beschreibung

Die Erfindung betrifft einen Extremitätendummy umfassend einen ersten und einen zweiten Dummyabschnitt, die mittels eines wenigstens eine Drehachse aufweisenden ersten Gelenkelementes gelenkig verbunden sind. Die Erfindung betrifft weiterhin ein Verfahren zum Testen der Funktion einer Orthese, die an eine Extremität, insbesondere eine untere Extremität eines Patienten anlegbar ist, insbesondere zur Ruhigstellung der Extremität.

Das Testen von Orthesen, z.B. Unterschenkelorthesen in Verbindung mit einem Extremitätendummy ist im Stand der Technik bekannt, z.B. aus der Publikation von D. Hochmann, "Prüfung von Unterschenkelorthesen", in ORTHOPÄDIE TECHNIK 05/14, Seite 102ff. Dort ist es beschrieben eine Unterschenkelorthese an einen Unterschenkeldummy anzulegen und mit einer Testvorrichtung Bewegungen gemäß EN ISO 22675 auf die Orthese auszuüben, die ein übliches Fußabrollen nachbilden. Zum Testen der Orthese wurden an der Orthese selbst auf Dehnungsmessstreifen basierende Sensoren in den Laminatschichten angebracht und so das Biegeverhalten der Orthese, sowie über die Zeit das Ermüdungsverhalten, bzw. das Nachgeben der Steifigkeit erfasst. Aus der Publikation von Wach, A., McGrady, L., Wang, M., and Silver-Thorn, B. (April 30, 2018), "Assessment of Mechanical Characteristics of Ankle-Foot Orthoses" ASME., J Biomech Eng. July 2018; 140(7): 071007 ist es auch bekannt, außen an der Orthese Marken anzubringen, mit denen eine Verschiebung der Orthese videobasiert ausgewertet wird. Ein Extremitätendummy nach dem Oberbegriff des Anspruchs 1 für das Testen einer Orthese ist bekannt aus den Publikationen von Amanda Watch "Mechanical Characterization of Carbon Fiber and Thermoplastic Ankle Foot Orthoses",MASTER'S THESES DISSERTATION, Bd. 341, 1. Dezember 2015 (2015-12-01), XP055581088, und Bregman D J J et AL "A new method for evaluating ankle foot orthosis characteristics: BRUCE",GAIT & POSTURE, ELSEVIER, AMSTERDAM, NL, Bd. 30, Nr. 2, 1. August 2009 (2009-08-01), Seiten 144-149, XP026237793,ISSN: 0966-6362, DOI: 10.1016/J.GAITPOST.2009.05.012 (gefunden am 2009-06-10).

Der verwendete Unterschenkeldummy umfasste einen Unterschenkelabschnitt und einen Fußabschnitt, die durch ein Gelenk verbunden sind. Der Fußabschnitt ermöglichte zudem ein Abrollen über den Vorderfuß im Zehengrundgelenk.

Eine Orthese ist ein medizinisches Hilfsmittel, insbesondere in der Art eines Exo-Skeletts, häufig einfach als Schiene bezeichnet, das eingesetzt wird z.B. für die Ruhigstellung oder auch nur zur Stabilisierung, Entlastung, oder Führung von Extremitäten oder des Rumpfes eines Patienten. Es ist dabei bekannt, dass Orthesen industriell oder in einem meist handwerklichen Prozess durch einen Orthopädietechniker hergestellt werden.

Die vorliegende Erfindung betrifft ebenso wie der genannte Stand der Technik das Anwendungsgebiet von Unterschenkelorthesen und Unterschenkeldummys ist jedoch nicht hierauf beschränkt. Grundsätzlich kann der erfindungsgemäße Extremitätendummy jegliche Extremität eines Patienten repräsentieren und die Orthese an jegliche solche Extremität anlegbar sein.

In dem bevorzugten Anwendungsgebiet ist die Erfindung besonders einsetzbar. Es ist beispielsweise bekannt, dass Patienten mit einer Diabetes-Erkrankung das diabetische Fuß-Syndrom entwickeln können, bei dem es bei einem schweren Verlauf zu einem Einbruch des Fußgewölbes kommen kann oder sogar Amputationen des Fußes durchgeführt werden müssen. Um dies zu verhindern stellen therapeutische Maßnahmen darauf ab, den betroffenen Fuß mittels einer an den Fuß und den Unterschenkel angelegten Orthese ruhigzustellen. Dabei kann von dem Grundsatz ausgegangen werden, dass der Therapieerfolg umso größer, ist je besser die Ruhigstellung durch die Orthese ist.

Problematisch ist in diesem Anwendungsgebiet, aber auch ganz allgemein, dass häufig industriell gefertigte Orthesen, die bei einer Vielzahl von Patienten möglichst gut passen sollen, keine genügende Funktion hinsichtlich der Ruhigstellung an der konkreten patientenindividuellen Extremität aufweisen.

Im benannten Anwendungsgebiet kann somit der Therapieerfolg trotz Tragen einer Orthese ausbleiben. Hier liegt die Vermutung nahe, dass patientenindividuell gefertigte Orthesen gegenüber industriell gefertigten Standard-Orthesen einen besseren Therapieerfolg mit sich bringen.

Aufgabe der Erfindung ist es einen Extremitätendummy und ein Verfahren zum Testen von Orthesen bereit zu stellen, mit denen sich die Möglichkeit ergibt die Funktion der Orthese hinsichtlich deren Wirkung an der Extremität besser als im Stand der Technik bewerten zu können. So bietet zwar der beschriebene Stand der Technik die Möglichkeit die Steifigkeit einer Orthese unter Last zu bewerten, ermöglicht aber nicht zu testen, welche Wirkungen die belastete Orthese auf die Extremität ausübt, kann also faktisch die Güte der Ruhestellung der Extremität nicht messtechnisch erfassen. Die Erfindung soll diese Möglichkeit bereitstellen. Erfindungsgemäß wird dies bei dem Extremitätendummy der eingangs genannten Art durch den gekennzeichneten Teil des Anspruchs 1 gelöst.

Mit einem solchen Dummy kann - im Gegensatz zu den aus den genannten Publikationen bekannten Dummy, die Lageänderung der Dummyabschnitte, z.B. Winkeländerungen oder ein absoluter Winkel zueinander gemessen werden, beispielsweise, wenn der Dummy eine Orthese trägt und diese belastet wird. Dies lässt Rückschlüsse zu auf die konkrete Wirksamkeit der Orthese zumindest am Dummy, was weiterhin Rückschlüsse zulässt auf die Wirksamkeit an der konkreten Extremität eines Patienten hinsichtlich der Ruhigstellung einer Extremität.

Es erschließt sich dadurch ein erfindungsgemäßes Verfahren zum Testen der Funktion einer Orthese, die an eine Extremität eines Patienten anlegbar ist, insbesondere zum Zweck der Ruhigstellung der Extremität, bei dem die zu testende Orthese an einen erfindungsgemäßen Extremitätendummy angelegt wird, der die Extremität des Patienten, an welche die Orthese anlegebar ist, repräsentiert, und auf wenigstens einen der Dummyabschnitte des Extremitätendummys eine Kraft ausgeübt wird, um diesen Dummyabschnitt gegenüber wenigstens einen anderen Dummyabschnitt, mit dem über ein Gelenkelement eine gelenkige Verbindung besteht, gegen die Wirkung der Orthese zu verlagern, und
mit wenigstens einem Sensor, der wenigstens einer Gelenkachse des Gelenkelementes zugeordnet ist, die Lage oder Lageänderung der Dummyabschnitte zueinander gemessen wird. Es kann dann beispielsweise vorgesehen sein, dass die Messwerte der Lage bzw. Lageänderung in Abhängigkeit der ausgeübten Kraft oder eines Lage-Parameters, vorzugsweise einer Winkelstellung, des die Kraft ausübenden Aktors erfasst wird. Anhand der erfassten Messwerte kann so nicht nur die Wirksamkeit der Orthese in sich, sondern die Wirksamkeit der Orthese am Extremitätendummy bzw. konkret an der Extremität des Patienten einfacher und zuverlässiger bewertet werden.

Besonders in der bevorzugten Anwendung bei der Therapie des diabetischen Fuß-Syndroms kann allgemein ausgesagt werden, dass die Wirkung einer getesteten Orthese besser ist, je kleiner die Lageänderungen bei Kraftausübung auf einen Dummyabschnitt des Extremitätendummys ist. Dies wird allgemein für alle Anwendungen einer Orthese gelten, in welcher diese zur Ruhigstellung von Extremitäten vorgesehen ist.

Bevorzugt ist es bei dem Extremitätendummy vorgesehen, dass dieser einen dritten Dummyabschnitt aufweist, der mit dem zweiten Dummyabschnitt mittels eines zweiten Gelenkelements, vorzugsweise eines nur eine Drehachse aufweisenden Gelenkelements gelenkig verbunden ist, wobei der wenigstens einen, vorzugsweise genau einen Drehachse des zweiten Gelenkelements ein Sensor zugeordnet ist, mit dem die Winkelstellung oder Winkeländerung der mit dem zweiten Gelenkelement verbundenen Dummyabschnitte bezüglich der zugeordneten Drehachse messbar ist.

Ein Extremitätendummy der Erfindung kann für die bevorzugte Anwendung einen Unterschenkeldummy ausbilden, wobei der erste Dummyabschnitt einen Unterschenkelabschnitt einer Unterschenkelextremität repräsentiert, und der zweite Dummyabschnitt einen Fußabschnitt, insbesondere einen Mittelfußabschnitt und/oder Rückfußabschnitt einer Unterschenkelextremität repräsentiert, und das erste Gelenkelement das Sprunggelenk, insbesondere oberes und unteres Sprunggelenk repräsentiert.

In der benannten bevorzugten Weiterbildung kann der dritte Dummyabschnitt einen Zehenabschnitt einer Fußextremität repräsentieren und das zweite Gelenkelement die Mittelfußgelenkreihe repräsentieren.

Bei den Gelenkelementen ist es vorzugsweise vorgesehen, dass diese die physiologische Funktion des repräsentierten Gelenkes nachbilden, insbesondere dadurch, dass die wenigstens eine Gelenkachse eines jeden Gelenkelements so orientiert ist, wie es bei einem Menschen, bzw. im Mittelwert bei einer Vielzahl von Menschen bei dem tatsächlichen Gelenk der Fall ist. Hier kann es auch vorgesehen sein, eine Beweglichkeit in einem physiologischen Gelenk des Menschen, insbesondere die komplexer ist als nur eine Achse, durch die Kopplung von wenigstens zwei Gelenkachsen im Gelenkelement des Extremitätendummys zu simulieren.

Bei dem ersten Gelenkelement, welches das Sprunggelenk repräsentiert kann vorgesehen sein, dass es eine erste Gelenkachse aufweist, die bei angenommener normal stehender Position des Extremitätendummys, die der stehenden Position eines Menschen entspricht, von medial nach lateral verläuft, insbesondere horizontal und senkrecht zur Transversalebene ausgerichtet ist. Statt einer horizontalen Ausrichtung kann auch bevorzugt eine Ausrichtung von 74 bis 94 Grad, vorzugsweise 82 Grad zur Sagittalebene vorgesehen sein, was mittleren Ausrichtungswerten entspricht, die an einer Vielzahl von Menschen erfasst sind. Diese Gelenkachse repräsentiert das obere Sprunggelenk.

Dasselbe Gelenkelement weist eine weitere Gelenkachse auf, die das untere Sprunggelenk repräsentiert. Diese Gelenkachse kann vorzugsweise in einem Winkelbereich von 4 bis 47 Grad, vorzugsweise 23 Grad zur Sagittalebene und im Bereich von 20 bis 69 Grad, vorzugsweise 42 Grad zur Horizontalebene orientiert sein.

Beim dem zweiten Gelenkelement, welches den Zehenabschnitt mit dem Mittelfuß und/oder Rückfußabschnitt verbindet, kann vorzugswiese eine einzige Gelenkachse vorgesehen sein, insbesondere die mittig im Zehenabschnitt, vorzugsweise in der Horizontalebene liegt, vorzugsweise die in einem Bereich von 53 Grad bis 73 Grad, insbesondere 62 Grad zur Sagittalebene geneigt ist. Mit einer solchen Achse kann die Beweglichkeit aller Zehen nachgebildet werden am Extremitätendummy der Erfindung das Abrollen des Fußes über die Zehen nachgebildet werden.

Allgemein kann zur Gelenkachsenausrichtung in den jeweiligen Gelenkelementen auf bestehende anatomische Daten zurückgegriffen werden.

Der Extremitätendummy kann vorzugsweise so ausgebildet sein, dass der den Unterschenkelabschnitt repräsentierende erste Dummyabschnitt an seinem oberen Ende einen Krafteinleitungsanschluß aufweist, mit dem eine Kraft in axialer und/oder radialer Richtung des Unterschenkelabschnittes in den Dummyabschnitt einleitbar ist, insbesondere welche die Gewichtskraft eines Patienten nachbildet, und/oder eine Kraft in den Dummyabschnitt einleitbar ist, mit dem der Dummyabschnitt um seine axiale Erstreckung tordierbar ist.

Dieser Krafteinleitungsanschluß weist vorzugsweise ein Gehäuseelement auf, das mit dem ersten Dummyabschnitt drehfest verbunden ist, in welchem ein Schaftelement drehbar gelagert ist, wobei ein Sensor vorgesehen ist, mit dem die Winkelstellung oder relative Verdrehung zwischen Schaftelement und Gehäuseelement messbar ist, vorzugsweise wobei das Schaftelement relativ zum Gehäuseelement zumindest zeitweise drehfest fixierbar oder zumindest in der Drehung hemmbar ist.

Im erfindungsgemäßem Testverfahren kann es vorgesehen sein, dass der Extremitätendummy vor oder nach dem Anlegen der Orthese in eine Testvorrichtung eingesetzt wird, wobei die Testvorrichtung wenigstens ein mit einem Aktor betätigbares Betätigungselement aufweist, mit dem auf wenigstens einen der Dummyabschnitte eine Kraft ausgeübt wird. Ein solches Vorgehen kann unabhängig von der bevorzugten Anwendung mit jeglicher Art von Extremitätendummy vorgesehen sein, um eine bestimmte Art von Orthese am Extremitätendummy auf ihre Funktion zu prüfen.

In der bevorzugten Anwendung, dass der Extremitätendummy eine Unterschenkelextremität repräsentiert und für Unterschenkel vorgesehene Orthesen zu testen sind kann es die Erfindung bevorzugt vorsehen, dass die Testvorrichtung ein Trittflächenelement aufweist, auf welches der wenigstens eine Dummyabschnitt mit der angelegten Orthese aufgestellt wird, der den Mittelfußbereich oder Rückfußbereich und/oder Zehenbereich repräsentiert und dass die Testvorrichtung ein Halteelement aufweist, mit dem eine haltende, tordierende oder eine Gewichtskraft simulierende Kraft auf das obere Ende des Dummyabschnitts ausgeübt wird, der den Unterschenkelabschnitt repräsentiert. Das obere Ende kann in diesem Fall z.B. der zuvor genannte Krafteinleitungsanschluss sein. Eine den Unterschenkelabschnitt in dessen axialer Erstreckung belastende Kraft kann z.B. auf den genannten Schaft ausgeübt werden. Eine den Unterschenkelabschnitt tordierende Kraft kann z.B. auf das Gehäuse des Krafteinleitungsanschlusses ausgeübt werden oder den drehgehemmten oder drehfixierten Schaft.

Bei einer ersten möglichen Testdurchführung kann es dann beispielsweise vorgesehen sein, dass das Trittflächenelement um eine zur Trittfläche parallele Achse, vorzugsweise horizontale Achse verschwenkt wird, insbesondere um ein Fußabrollen beim Gehen zu simulieren. Durch das Trittflächenelement werden Kräfte auf die Orthese und über diese auf den Extremitätendummy ausgeübt, die üblicherweise beim Gehen eines Patienten auftreten können.

In einer zweiten möglichen Art der Testdurchführung kann es vorgesehen sein, dass das Trittflächenelement vor einer Krafteinleitung auf das obere Ende des Dummyabschnitts, der den Unterschenkelabschnitt repräsentiert, in einer eine vorbestimmte Phase der Fußabrollung beim Gehen entsprechenden Winkel-Lage fixiert wird, und anschließend das obere Ende des Dummyabschnitts, der den Unterschenkel repräsentiert, mit einer Kraft belastet wird, insbesondere der Gewichtskraft eines Patienten oder einer für Menschen üblichen mittleren Gewichtskraft. So können Kräfte auf die Orthese und den Extremitätendummy in verschiedenen Phasen des Gehens gezielt ausgeübt werden.

Hierbei ist es auch denkbar, über den drehbaren Krafteinleitungspunkt den Fuß, gemäß der natürlichen Stellung des Fußes, um einen gewissen Winkel nach außen zu verdrehen, um dann eine funktionale Prüfung durchzuführen.

Eine weitere dritte Art der Testdurchführung kann es vorsehen, dass der den Mittelfußbereich oder Rückfußbereich und/oder Zehenbereich repräsentierende Dummyabschnitt auf dem Trittflächenelement mittelbar über die Orthese drehfest um die Längsachse des Unterschenkelabschnittes fixiert wird und auf den Dummyabschnitt, der den Unterschenkelabschnitt repräsentiert, eine diesen um dessen Längsachse tordierende Kraft ausgeübt wird. Aufgrund der kardanähnlichen Verbindung zwischen Unterschenkelabschnitt und Mittelfuß / Rückfußabschnitt kann es im Bereich des Fußes zu einem Verkippen bei Torsion des Unterschenkels kommen, was besonders bei dem diabetischen Fußsyndrom zu vermeiden ist und deswegen ein besonders wichtiges Testkriterium darstellen kann.

Ebenso sind weitere Arten der Testdurchführung möglich, die hier nicht abschließend beschrieben sind.

Bei allen möglichen Testdurchführungen kann es die Erfindung vorsehen, dass die Winkelstellung und/oder Winkeländerung wenigstens eines Dummyabschnittes gegenüber einem anderen, mit dem wenigstens einen Sensor in dem diese Dummyabschnitte verbindenden Gelenkelement gemessen wird, insbesondere in Abhängigkeit der aufgewendeten Kraft und/oder in Abhängigkeit eines Lage-Parameters, vorzugsweise einer Winkelstellung, des die Kraft ausübenden Aktors. Z.B. kann hier die Winkelstellung des Trittflächenelementes erfasst werden. Die aufgewendete Kraft kann z.B. die mit dem Trittflächenelement erzeugte Kraft sein und/oder die in das Krafteinleitungselement des Unterschenkelabschnitts eingeleitete Kraft, welche z.B. die Gewichtskraft eines Patienten simulieren kann. Die jeweiligen Sensoren können mit einer Messelektronik verbunden sein, um die Meßwerte zu erfassen und zu speichern, insbesondere in Abhängigkeit der genannten Kraft oder des Lage-Parameters. Die Messelektronik kann ebenso die Ansteuerung des wenigstens einen Aktors zur Ausübung einer Kraft oder Verstellung des Trittflächenelementes vornehmen.

Sowohl der Extremitätendummy als auch die Orthese kann hinsichtlich der Konstruktion als ein jeweiliges Standard-Element ausgeführt sein, insbesondere welches eine möglichst gute jeweilige Anpassung an die Anatomie zu einer Vielzahl von Menschen aufweist. Das kann z.B. erzielt werden, wenn die Orthese und/oder der Extremitätendummy anhand der gemittelten anatomischen Daten einer Vielzahl von Menschen angefertigt werden.

In bevorzugter Ausführung kann es die Erfindung vorsehen bei der Orthese und/oder bei dem Extremitätendummy die tatsächlichen anatomischen Verhältnisse desjenigen Patienten zu berücksichtigen, für den die Orthese vorgesehen ist. Es kann so mit verbesserter Genauigkeit die Wirksamkeit der Orthese an der tatsächlichen Extremität des Patienten zuvor im Testverfahren ermittelt werden.

Es kann bevorzugt vorgesehen sein, dass wenigstens einer von mehreren Dummyabschnitten, vorzugsweise jeder Dummyabschnitt zumindest bereichsweise, insbesondere zumindest in solchen Bereichen, die von einer Orthese kontaktierbar sind, vorzugsweise vollständig, in Abhängigkeit, vorzugsweise entsprechend der individuellen Form des zu dem Dummyabschnitt korrespondierenden Abschnittes oder des zu dem Bereich des Dummyabschnitts korrespondierenden Bereiches an der Extremität eines Patienten ausgebildet ist.

Hierdurch wird eine verbesserte Anpassung des Extremitätendummys an die tatsächliche Körperextremität des späteren Orthesenträgers erzielt.

Ein Extremitätendummy, der in Abhängigkeit der individuellen Form des zu dem Dummyabschnitt korrespondierenden Abschnittes an der Extremität eines Patienten ausgebildet ist, kann z.B. so hergestellt sein, dass der Extremitätendummy aus mehreren Dummyabschnitten zusammengesetzt ist, wobei jeder zu nutzende Dummyabschnitt aus einem Satz von Dummyabschnitten ausgewählt wird, insbesondere die alle denselben Extremitätenabschnitt repräsentieren und in jedem Satz Dummyabschnitte unterschiedlicher Größe oder Art vorliegen.

Z.B. kann es einen Satz von Unterschenkelabschnitten geben in dem insoweit verschiedene Größen oder Arten vorliegen, dass die Unterschenkelabschnitte z.B. verschiedene Körpergrößen oder Größenstufen (z.B. S, M, L, XL) repräsentieren oder verschieden starke Wadenmuskulatur oder ähnliches. Bei den Mittelfußdummyabschnitten können in einem Satz z.B. verschiedene Fußgrößen (Schuhgrößen) vorliegen und/oder verschiedene Fußformen, wie z.B. zur Berücksichtigung von Spreizfuß, Plattfuß, Hohlfuß etc.

Nach Auswahl der für den konkreten Patienten am besten passenden Dummyabschnitte aus einem jeweiligen Extremitätenabschnitt-Satz, können die Dummyabschnitte verbunden werden, vorzugsweise mit den Gelenkelementen, z.B. durch Verschraubung.

Bei dem vorbeschriebenen Vorgehen wird zwar eine verbesserte Individualisierung in Abhängigkeit der Anatomie des Patienten erzielt, aber noch nicht eine Individualisierung entsprechend der Anatomie.

Die Erfindung kann es beispielsweise vorsehen, dass wenigstens einer der Dummyabschnitte des Extremitätendummys, vorzugsweise jeder Dummyabschnitt so ausgebildet, insbesondere geformt wird, dass dessen Form zumindest bereichsweise, insbesondere insgesamt, ganz konkret derjenigen Form entspricht, die der korrespondierende Abschnitt / Bereich an der tatsächlichen Extremität des Patienten aufweist. Der jeweils form korrespondierende Dummyabschnitt des erfindungsgemäßen Extremitätendummys ist somit vorzugsweise zumindest bereichsweise form identisch zum korrespondierenden Abschnitt an der Extremität des Patienten.

Auch hier werden nach Anfertigung der Dummyabschnitte für den konkreten Patienten die Dummyabschnitte miteinander verbunden, um den gesamten Extremitätendummy zu bilden, vorzugsweise mit den Gelenkelementen, z.B. durch Verschraubung.

Es kann in allen möglichen Ausführungen der Individualisierung des Extremitätendummys ebenso vorgesehen sein, die Gelenkelemente jeweils aus Sätzen mit mehreren verschiedenen Gelenkelementen zu wählen, um auch bei den Gelenkelementen einen Grad der Individualisierung zu vergrößern. Die Individualisierung kann z.B. hier bzgl. der Orientierung der Gelenkachsen erfolgen.

Eine weitere Möglichkeit eine verbesserte Individualisierung zu erzielen kann vorsehen, dass wenigstens einer der Dummyabschnitte des Extremitätendummys, vorzugsweise jeder Dummyabschnitt einen Befestigungsbereich aufweist, auf den wenigstens ein Formelement, vorzugsweise nebeneinander mehrere Formelemente befestigbar sind, wobei das jeweilige Formelement entweder entsprechend der individuellen Form des zu dem Formelement korrespondierenden Bereiches an der Extremität eines Patienten ausgebildet ist oder aus einem Satz von mehreren größenverschiedenen oder artverschiedenen Formelementen gewählt ist. Z.B. kann ein Satz mehrere Formelemente mit stufenweise verschiedenen Größen aufweisen.

Ein Befestigungsbereich kann z.B. als ein Holm ausgebildet sein, vorzugsweise, der sich zwischen den in Längsrichtung des Dummyabschnittes liegenden Enden desselben erstreckt, z.B. zwei Gelenkelemente verbindet oder ein Gelenkelement mit einem Krafteinleitungselement verbindet. Auf einen solchen Holm können sodann die Formelemente aufgesetzt werden, vorzugsweise verdrehsicher, insbesondere form- und / oder kraftschlüssig. Vorzugsweise ist der Holm mit einem nicht kreisförmigen Querschnitt ausgebildet, vorzugsweise mit mehreckigem Querschnitt.

Ein jeweiliges Formelement kann einteilig ausgebildet sein. Z.B. erstreckt es sich dann in Umfangsrichtung um den Holm um weniger als 360 Grad, vorzugsweise aber mehr als 270 Grad, um eine Öffnung zum Aufsetzen auf den Holm bereitzustellen. Es kann auch vorgesehen sein, dass ein Formelement zweiteilig oder mehrteilig ausgebildet ist, vorzugsweise im zusammengebauten Zustand über den Umfang geschlossen ist. Im geteilten Zustand kann sodann das Formelement auf dem Holm befestigt und zusammengefügt werden, vorzugsweise hiernach den Holm vollständig umgeben.

Mit Bezug auf die vorgenannten Ausführungsformen, kann die Erfindung z.B. vorsehen, dass ein jeweiliger formkorrespondierender Dummyabschnitt oder ein jeweiliges formkorrespondierendes Formelement ausgebildet ist als ein generativ, insbesondere durch 3D-Druck, hergestelltes Bauteil in Abhängigkeit von den korrespondierenden Abschnitt an der Extremität eines Patienten repräsentierenden Daten.

Es kann ebenso vorgesehen sein, dass ein jeweiliger formkorrespondierender Dummyabschnitt oder ein jeweiliges formkorrespondierendes Formelement ausgebildet ist als spanend aus einem Rohling hergestelltes Bauteil in Abhängigkeit von den korrespondierenden Abschnitt an der Extremität eines Patienten repräsentierenden Daten.

Solche Daten für die beiden vorgenannten Ausführungen können z.B. durch einen optischen Scan der Extremität erfasst werden, z.B. durch einen Laserscan.

Weiterhin kann es ebenso vorgesehen sein, dass ein jeweiliger formkorrespondierender Dummyabschnitt oder ein jeweiliges formkorrespondierendes Formelement ausgebildet ist als Bauteil, dass von einer den korrespondierenden Abschnitt an der Extremität eines Patienten entsprechenden Negativform abgeformt ist. Eine solche Negativform kann z.B. ein Abdruck der Patientenextremität mit einer Abdruckmasse, z.B. ein Gipsabdruck bilden, der anschließend ausgegossen wird, um den Dummyabschnitt oder das Formelement zu bilden.

Besonders die formkorrespondierende Ausbildung des Extremitätendummys in wenigstens einem Dummyabschnitt, vorzugsweise allen Dummyabschnitten bietet die beste Möglichkeit, die Wirksamkeit einer Orthese zu testen, insbesondere wenn diese selbst an die Anatomie, also die Form der Extremität des Patienten angepasst ist, an welche die Orthese anzulegen ist.

Vorzugsweise kann es vorgesehen sein, dass der Extremitätendummy nicht nur formkorrespondierend zur Extremität eines Patienten ausgebildet wird, sondern auch in Abhängigkeit der für denselben Patienten individuell ausgebildeten Orthese.

So wird eine Orthese beispielsweise nicht vollständig eine Extremität des Patienten umschließen, z.B. schon nicht aus dem Grund, um diese an die Extremität anlegen zu können oder um eine genügende Belüftung zu bieten. Es gibt also in einer Orthese Bereiche, die nicht an der Extremität anliegen bzw. wo die Orthese offen ist. In solchen Bereichen ist die Orthese zwangsläufig nicht an die Extremität des Patienten angepasst. Die Erfindung kann somit vorsehen, dass der Extremitätendummy in solchen Bereichen, wo die Orthese keine formkorrespondierende Ausbildung zur Extremität des Patienten hat, der Extremitätendummy in seinen Dummyabschnitten oder Formelementen ebenso nicht formkorrespondierend ausgebildet ist.

Eine weitere Ausführung kann es vorsehen, dass verschiedene Dummyabschnitte und/oder verschiedene Bereiche eines Dummyabschnittes oder verschiedene Formelemente verschiedene Verformbarkeit oder Steifigkeit aufweisen, insbesondere durch Ausbildung aus Materialien mit verschiedenem E-Modul oder Ausbildung mit verschiedenen Versteifungsstrukturen, und/oder verschiedene Oberflächeneigenschaften aufweisen, insbesondere durch unterschiedliche Oberflächenbeschichtungen. Z.B. kann die Oberfläche eines Dummyabschnitts oder Formelements mit einem Textil überzogen werden, insbesondere um die Reibung zwischen Orthese und Haut besser zu simulieren oder um die Verschieblichkeit der Hautoberfläche nachzubilden.

Auch hierdurch kann eine Anpassung an verschiedene anatomische Gegebenheiten beim Patienten erfolgen.

Weiterhin kann es die Erfindung vorsehen, dass sich gegenüberliegende Gelenkelemente eines Dummyabschnitts oder sich gegenüberliegender Krafteinleitungsanschluß und Gelenkelement eines Dummyabschnitts mittels eines Versteifungselementes verbunden sind, welches eine höhere Steifigkeit aufweist, als das Material des Dummyabschnitts, welches das Versteifungselement umgibt.

Ein solches Versteifungselement kann gleichzeitig den zuvor benannten Befestigungsbereich, z.B. einen Holm zur Befestigung von wenigstens einem Formelement bilden.

Mit dem vorbeschriebenen Extremitätendummy erschließt sich im besonders bevorzugten Verfahren, dass der Extremitätendummy zumindest bereichsweise an die Extremität eines Patienten individuell angepasst ist/wird, so dass der Extremitätendummy in den angepassten Bereichen der Form der Extremität des Patienten entspricht, insbesondere wobei auch die zu testende Orthese zumindest bereichsweise an die Extremität eines Patienten individuell angepasst ist/wird, insbesondere durch zumindest bereichsweise Ausbildung der Orthese als Negativform, die zur Form der Extremität des Patienten korrespondiert, und die Orthese, insbesondere die angepasste Orthese an den angepassten Extremitätendummy angelegt wird. Vorzugsweise wird dabei der Extremitätendummy nur in denjenigen Bereichen an die Extremität eines Patienten individuell angepasst, in denen auch die Orthese angepasst wird.

In diesem Fall spiegelt der Test der angepassten Orthese am angepassten Extremitätendummy besonders gut das spätere Verhalten der Orthese an der Extremität des Patienten wieder. Die funktionale Wirksamkeit der Orthese hinsichtlich einer Ruhigstellung von Extremitäten kann so besonders zuverlässig geprüft oder bestätigt werden, insbesondere wenn durch die Anpassung der Dummyabschnitte im E-Modul seines Materials und evtl. Oberflächenbeschichtungen an die Extremität des Patienten auch das Weichteilverhalten der Extremität des Patienten nachgebildet wird.

Ausführungsbeispiele der Erfindung werden nachfolgend beschrieben.

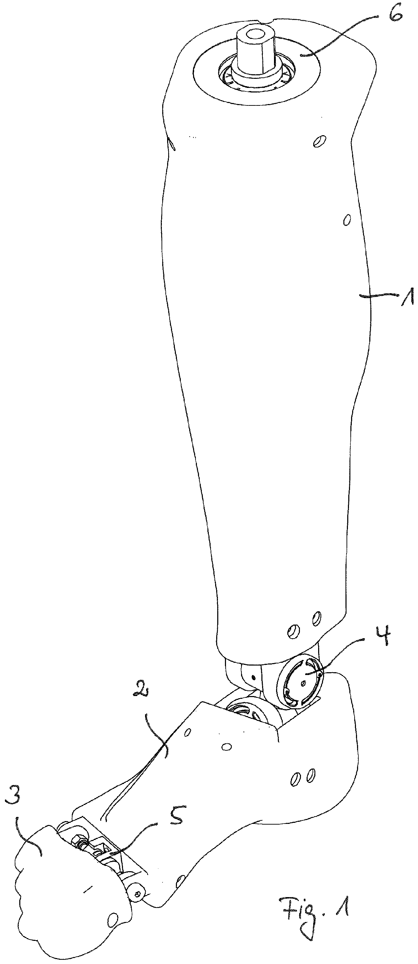

Die Figur 1 zeigt eine Gesamtübersicht eines Extremitätendummys der Erfindung, der in diesem Beispiel einen Unterschenkel repräsentiert. Erweist einen ersten Dummyabschnitt 1 auf, der den Unterschenkelabschnitt bildet, einen zweiten Dummyabschnitt 2, der den Mittelfußabschnitt bildet, und einen dritten Dummyabschnitt 3 auf, der den Zehenabschnitt eines Fußes bildet.

Der erste und zweite Dummyabschnitt 1, 2 sind mit einem ersten Gelenkelement 4 gelenkig verbunden. Der zweite und dritte Dummyabschnitt 2, 3 sind mit einem zweiten Gelenkelement 5 verbunden. Am oberen Ende des ersten Dummyabschnittes 1 ist ein Krafteinleitungselement 6 vorgesehen.

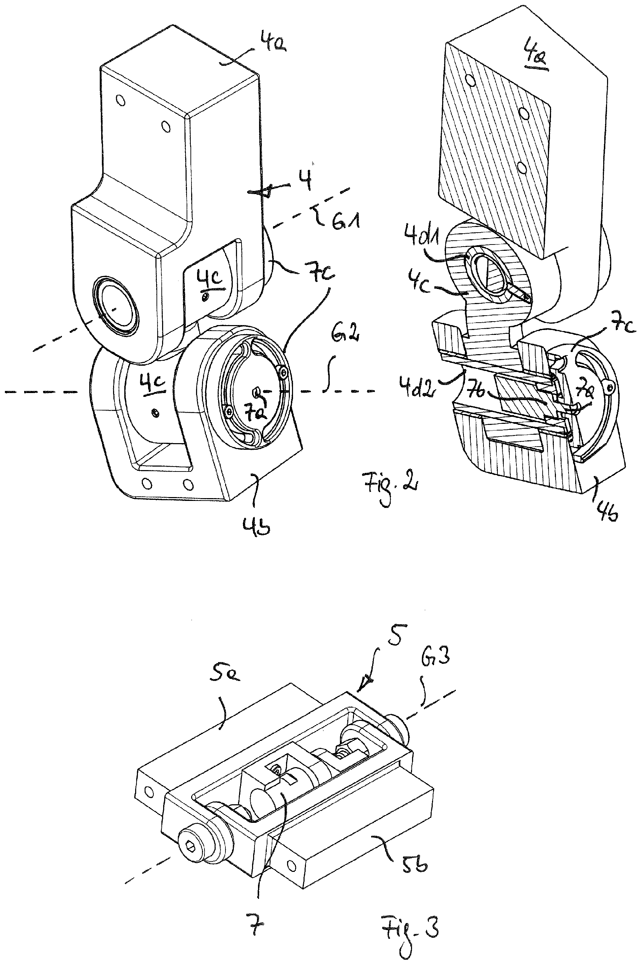

In den Figuren 2 bis 4 sind die Elemente 4, 5 und 6 genauer dargestellt. Das erste Gelenkelement 4 gemäß Figur 2 weist eine erste Gelenkachse G1 und eine zweite Gelenkachse G2 auf. Durch diese beiden Gelenkachsen wird die Funktion des oberen und unteren Sprunggelenkes des Menschen simuliert.

Das zweite Gelenkelement 5 gemäß Figur 3 weist hier nur eine Gelenkachse G3 auf, um die Zehenbeweglichkeit zu simulieren.

Die Achsausrichtung der Gelenkachsen kann jeweils so vorgenommen sein, wie im allgemeinen Beschreibungsteil genannt.

Jedes Gelenkelement 4, 5 weist für jede seiner Gelenkachsen einen Sensor 7 auf, mit dem die Winkelstellung oder Winkeländerung um die jeweilige Gelenkachse gemessen und messtechnisch erfasst werden kann. Solche Sensoren können z.B. mit Hall-Effekt, über die Potentialänderung der Spannung auf die jeweilige Winkelstellung schließen. Hierzu bedarf es einer kabelgebundenen elektronischen Spannungsversorgung der Sensoren. Ein geeigneter Sensor ist z.B. aus der Serie Euro-CMRT der Firma Variohm.

Beim Gelenkelement 5 ist der Stator des Sensors 7 mit einem der um die Gelenkachse herum angeordneten Gelenkelementteile verbunden, der Rotor des Sensors mit dem jeweilig anderen Gelenkelementteil.

Das Sprunggelenk umfasst beispielsweise die beiden Gelenkelementteile 4a, 4b sowie ein die beiden Gelenkelementteile 4a, 4b verbindendes Gelenkelementteil 4c. Das verbindende Gelenkelementteil 4c ist drehfest mit einer jeweiligen Hohlwelle 4d1 bzw. 4d2 um die jeweilige Drehachse G1 und G2 verbunden. In jeder der beiden Hohlwellen 4d1 und 4d2 ist ein jeweiliger Sensor 7 angeordnet. Dabei ist der Stator 7b des Sensors 7 drehfest an der Hohlwelle 4d1 bzw. 4d2 befestigt, z.B. mit einer Gewindeverbindung. Der Rotor 7a liegt mit seinem Ende drehfest, z.B. formschlüssig in einem scheibenförmigen Stellelement 7c ein, mit dem die Winkelstellung des Rotors 7a beeinflußbar ist, z.B. um eine Referenzstellung oder Neutralstellung einzustellen, und welches drehfest befestigbar ist am Gelenkelementteil 4a bzw. 4b. Somit werden demnach die Erfassung der Winkelstellungen bzw. Änderungen zwischen den Gelenkelementteilen 4a und 4c bzw. 4b und 4c ermöglicht.

Solche Sensoren können z.B. Winkelencoder sein, die an eine Messelektronik angeschlossen sind.

Jedes Gelenkelement 4, 5 weist zwei durch die Drehachsen oder die Drehachse herum angeordnete Gelenkelementteile 4a, 4b bzw. 5a, 5b auf, die mit den um das jeweilige Gelenkelement 4, 5 herumliegenden Dummyabschnitten verbunden werden, z.B. durch Verschraubung.

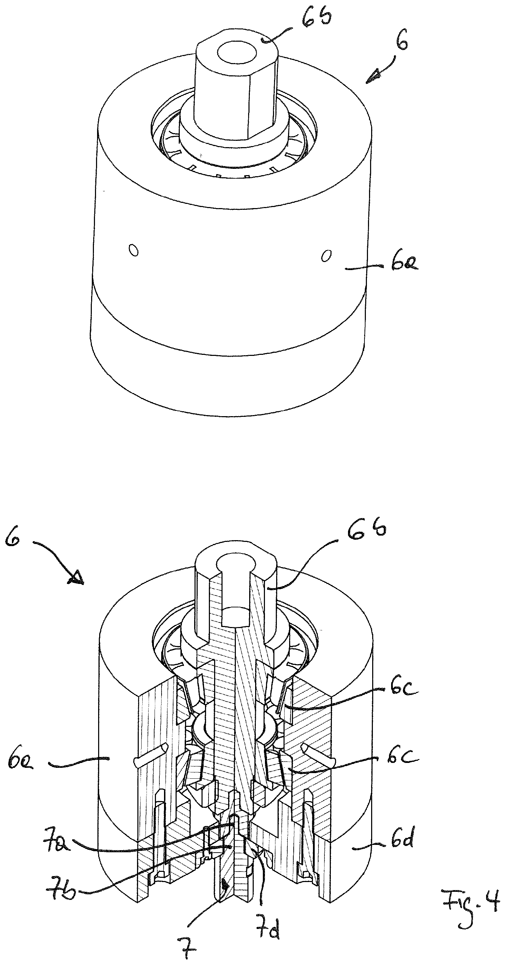

Das in der Figur 4 gezeigte Krafteinleitungselement 6 weist ein äußeres Gehäuse 6a auf, in dem ein Schaft 6b rotierbar gelagert ist, z.B. mittels zwei Kugellagern oder Zylinderrollenlagern 6c. Hier unten am Gehäuse 6a ist ebenso ein Sensor 7 angeordnet, mit dem die Winkelstellung oder Änderung des Schaftes 6b im Gehäuse 6a messbar ist. Das Gehäuse 6a wird am Dummyabschnitt drehfest befestigt, z.B. verschraubt, der den Unterschenkel repräsentiert. Hier kann der Rotor 7a des Sensors mit dem Schaft 6b drehfest verbunden sein und der Stator 7b mit dem Gehäuse 6a mittelbar über den Statorflansch 7d und den Befestigungsring 6d des Gehäuses 6a

Durch eine Kraft, die axial oder auch in einem Winkel ungleich 0 Grad zur Schaftachse auf den Schaft 6b wirkt, kann eine Gewichtskraftbelastung des Extremitätendummy simuliert werden, z.B. durch die Gewichtskraft des Patienten, der eine zu testende Orthese später tragen soll. Es kann auch vorgesehen sein, eine Kraft größer als diese Gewichtskraft auszuüben.

Durch eine Kraft, die in Umfangsrichtung um die Drehachse des Schaftes 6b am Gehäuse 6a angreift, kann eine Torsion des Unterschenkelabschnittes erzielt werden. Diese Kraft wirkt vorzugsweise auf das Gehäuse 6a direkt oder mittelbar auf mit dem Gehäuse 6a verbundene Elemente.

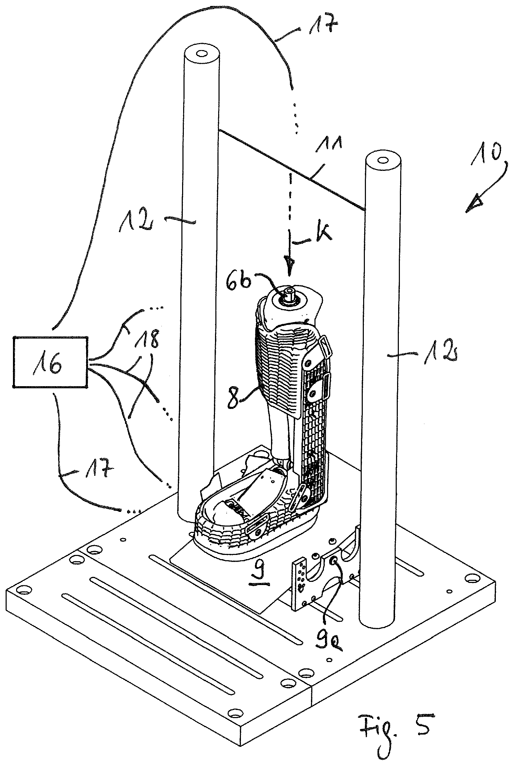

Die Figur 5 zeigt auszugsweise eine Testvorrichtung 10, in die ein Extremitätendummy mit angelegter Orthese 8 eingesetzt ist, so dass die Dummyabschnitte 2 und 3, welche Mittelfuß und Zehen repräsentieren über die Orthese 8 auf einem Trittflächenelement 9 der Testvorrichtung 10 aufliegen. Das Trittflächenelement 9 ist in dieser Ausführung in zwei Lagerböcken um die horizontale Schwenkachse 9a verschwenkbar gelagert. Die Verschwenkung kann z.B. mit einem nicht gezeigten Aktor erfolgen. Der Aktor kann z.B. von einer Messvorrichtung 16 angesteuert werden, mit welcher auch die Messwerte der Sensoren 7 erfasst und gespeichert werden. Wie in der Figur 5 gezeigt kann dafür die Messvorrichtung 16 mit Steuerkabeln 17 mit den Aktoren verbunden sein, um das Trittflächenelement (9) zu bewegen und/oder die Kraft K zu erzeugen. Mit Messkabeln 18 können die Messwerte von den Sensoren 7 zur Messvorrichtung 16 übertragen werden. Dies ist nur in der Figur 5 visualisiert, gilt aber für die anderen Figuren ebenso.

Damit der Extremitätendummy in der Testvorrichtung 10 sicher gehalten ist, greift eine hier nur symbolisch gezeigte Haltevorrichtung 11, die an den Säulen 12 befestigt sein kann, am oberen Ende des Extremitätendummys an und übt eine haltende und/oder Gewichtskraft ausübende Kraft K auf den Schaft 6b aus. Die Kraftausübung auf den Schaft 6b kann in diesem Beispiel bereits in Richtung der Schaftachse erfolgen.

Ein Test kann nun so durchgeführt werden, dass z.B. unter der Wirkung der Kraft K das Trittflächenelement 9 in seinem Winkel um die Drehachse 9a kontinuierlich in einem vorbestimmten Winkelbereich verstellt wird.

Es kann alternativ auch eine vorbestimmte feste Winkelstellung des Trittflächenelementes 9 vorgegeben und hiernach der Extremitätendummy mit einer Kraft K auf das Krafteinleitungselement 6 belastet werden. Die Winkelstellung wird vorzugsweise über die Gelenkachse 9a verstellt.

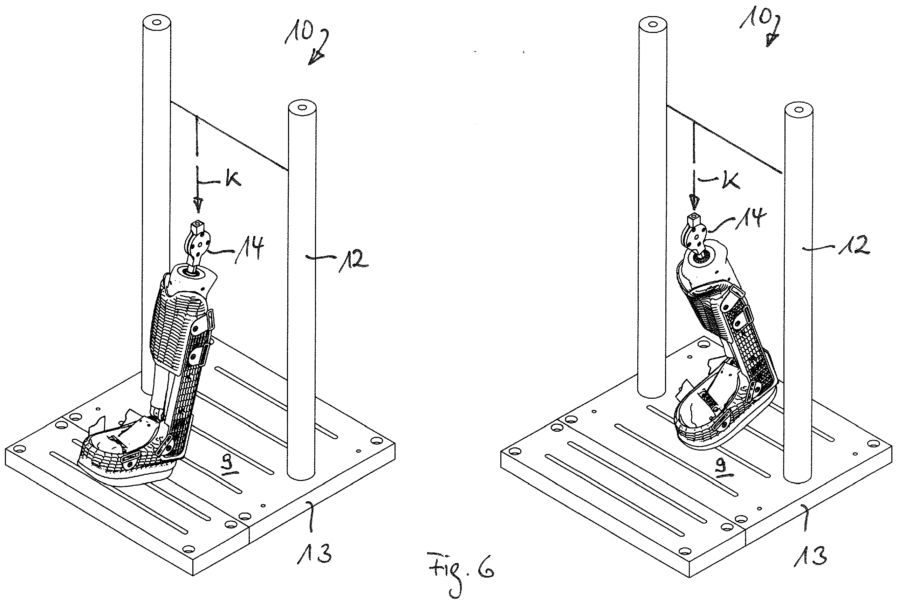

Gemäß Figur 6 kann es auch vorgesehen sein, dass das verschwenkbare Trittflächenelement 9 entfällt und ein festes Trittflächenelement durch die Basisplatte 13 der Testvorrichtung 10 gebildet wird. In dieser Ausführung ist es vorgesehen, die Kraft K, die auf den Schaft 6b des Krafteinleitungselementes wirkt, mittelbar über ein Gelenkelement 14 in den Schaft 6b einzuleiten. Diese Gelenkelement 14 kann die Kniegelenkfunktion in z.B. einer Achse simulieren.

Es kann dann vorgesehen sein, die Kraft mittelbar über das Gelenkelement 14 auf das obere Ende des Dummyabschnittes auszuüben, insbesondere auf den Schaft 6b des Krafteinleitungselementes 6, während die Gelenkstellung des Gelenkelements 14 stufenweise oder kontinuierlich verstellt wird oder die Gelenkstellung vor der Krafteinleitung auf das obere Ende des Dummyabschnitts, der den Unterschenkelabschnitt repräsentiert, in einer eine vorbestimmte Phase der Fußabrollung beim Gehen entsprechenden Winkel-Lage fixiert wird, und anschließend das obere Ende mit einer Kraft belastet wird.

Eine Ausbildung kann vorsehen, dass bei der Anordnung der Figur 6 mit einem Aktor das Gelenkelement 14 in Gehrichtung verlagert wird, um so eine Gehbewegung zu simulieren, insbesondere während einer Kraftausübung.

Bei diesen Testversionen wird der Winkel oder die Winkeländerung in zumindest einer der Drehachsen, vorzugsweise aller Drehachsen der Gelenkelemente 4, 5 ggfs. auch im Krafteinleitungselement 6 mit den Sensoren 7 erfasst und mit einer Messelektronik aufgezeichnet, vorzugsweise in Abhängigkeit der Winkelstellung des Trittflächenelementes und/oder der wirkenden Kraft K.

Allgemein kann in allen möglichen Ausführungen die Messelektronik z.B. über Kabel mit den Sensoren 7 verbunden sein. Auch kabellose Sensoren mit einer Funkverbindung zu der Messelektronik können eingesetzt werden.

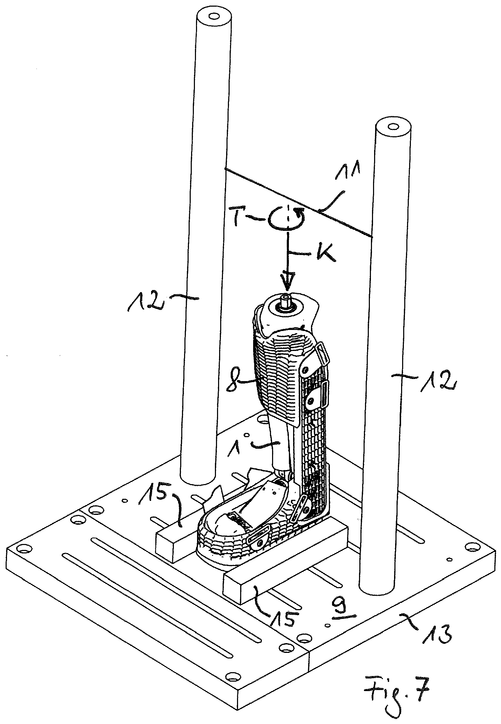

Bei der Figur 7 ist das Extremitätendummy oben so wie in der Figur 5 gehalten und mit einer Kraft K belastet. Das Trittflächenelement 9 ist in diesem Fall wieder durch die Basisplatte 13 der Testvorrichtung 10 gebildet und nicht verschwenkbar. Auf diesem Trittflächenelement 9 ist der Extremitätendummy zusammen mit der angelegten Orthese 8 durch die seitlichen Backenelemente 15 gegen ein Verdrehen, insbesondere bzgl. der Längsachse des Unterschenkelabschnittes 1, fixiert.

Es kann nun um die Unterschenkellängsachse, die der Richtung der Kraft K entsprechen kann, eine Torsionskraft T auf das obere Ende des Unterschenkelabschnitts 1 des Extremitätendummy ausgeübt werden, z.B. durch einen kraftbelasteten Hebel, der am Gehäuse 6a des Krafteinleitungselementes 6 angreift.

Es wird sodann zumindest in den Drehachsen G1 und G2 des Gelenkelementes 4 gemessen, welcher Winkel bzw. welche Winkeländerung sich in Abhängigkeit der Torsionskraft T und/oder der Kraft K ergibt.

Bei einer Anordnung des Extremitätendummys mit Orthese 8 auf einer verschwenkbaren Trittfläche 9 gemäß Figur 1 könnte derselbe Test auch für verschiedene Schwenkwinkel des Trittflächenelementes 9 durchgeführt werden.

Es kann mit den beispielhaft gezeigten Ausführungen eine bestimmte Orthese 8 in Verbindung mit einem Extremitätendummy hinsichtlich Ihrer funktionalen Wirkung bezüglich der Ruhigstellung einer Extremität getestet werden. Vorzugsweise können industriell gefertigte Standardorthesen in der Wirkung mit patientenindividuell gefertigten Orthesen 8 verglichen werden, besonders, wenn auch der Extremitätendummy an den Patienten angepasst ist, wie es eingangs beschrieben wurde.

## Patentansprüche

1. Extremitätendummy, der einen Unterschenkeldummy (1, 2, 3, 4, 5) bildet, umfassend einen ersten (1) und einen zweiten (2) Dummyabschnitt (1, 2), die mittels eines wenigstens eine Drehachse (G1, G2) aufweisenden ersten Gelenkelementes (4), welches das Sprunggelenk repräsentiert, gelenkig verbunden sind, wobei
a. der erste Dummyabschnitt (1) einen Unterschenkelabschnitt (1) einer Unterschenkelextremität repräsentiert, und
b. der zweite Dummyabschnitt (2) einen Fußabschnitt, insbesondere einen Mittelfußabschnitt (2) und/oder Rückfußabschnitt einer Unterschenkelextremität repräsentiert,
**dadurch gekennzeichnet, dass** das erste Gelenkelement das obere und das untere Sprunggelenk repräsentiert und die Beweglichkeit im physiologischen Gelenk des Menschen durch Kopplung von wenigstens zwei Gelenkachsen im ersten Gelenkelement simulierbar ist, wobei jeder der mehreren Drehachsen (G1, G2) des Gelenkelements (4) ein Sensor (7) zugeordnet ist, mit dem die Winkelstellung oder Winkeländerung der verbundenen Dummyabschnitte (1, 2) bezüglich der zugeordneten Drehachse (G1, G2) messbar ist und der den Unterschenkelabschnitt repräsentierende erste Dummyabschnitt (1) an seinem oberen Ende einen Krafteinleitungsanschluß (6) aufweist, über den eine Kraft (T) in den Dummyabschnitt (1) einleitbar ist, mit dem der Dummyabschnitt (1) um seine axiale Erstreckung tordierbar ist.

2. Extremitätendummy nach Anspruch 1, **dadurch gekennzeichnet, dass** ein dritter Dummyabschnitt (3) vorgesehen ist, der mit dem zweiten Dummyabschnitt (2) mittels eines zweiten Gelenkelements (5), vorzugsweise eines nur eine Drehachse (G3) aufweisenden Gelenkelements (5) gelenkig verbunden ist, wobei der wenigstens einen, vorzugsweise genau einen Drehachse (G3) des zweiten Gelenkelements (5) ein Sensor (7) zugeordnet ist, mit dem die Winkelstellung oder Winkeländerung der mit dem zweiten Gelenkelement (5) verbundenen Dummyabschnitte (2, 3) bezüglich der zugeordneten Drehachse (G3) messbar ist.

3. Extremitätendummy nach Anspruch 2, **dadurch gekennzeichnet, dass** der dritte Dummyabschnitt (3) einen Zehenabschnitt einer Unterschenkelextremität repräsentiert und das zweite Gelenkelement (5) die Mittelfußgelenkreihe repräsentiert.

4. Extremitätendummy nach einem der vorherigen Ansprüche, **dadurch gekennzeichnet, dass** wenigstens einer von mehreren Dummyabschnitten (1, 2, 3), vorzugsweise jeder Dummyabschnitt (1, 2, 3) zumindest bereichsweise, insbesondere zumindest in solchen Bereichen, die von einer Orthese (8) kontaktierbar sind, vorzugsweise vollständig, in Abhängigkeit, vorzugsweise entsprechend der individuellen Form des zu dem Dummyabschnitt (1, 2, 3) korrespondierenden Abschnittes oder des zu dem Bereich des Dummyabschnitts (1, 2, 3) korrespondierenden Bereiches an der Extremität eines Patienten ausgebildet ist.

5. Extremitätendummy nach Anspruch 4, **dadurch gekennzeichnet, dass** der Extremitätendummy (1, 2, 3, 4, 5) aus mehreren Dummyabschnitten (1, 2, 3) zusammengesetzt ist, wobei jeder zu nutzende Dummyabschnitt (1, 2, 3) aus einem Satz von Dummyabschnitten (1, 2, 3) auswählbar ist, insbesondere die alle denselben Extremitätenabschnitt repräsentieren und in jedem Satz Dummyabschnitte (1, 2, 3) unterschiedlicher Größe und/oder Art vorliegen.

6. Extremitätendummy nach Anspruch 4, **dadurch gekennzeichnet, dass** wenigstens einer der Dummyabschnitte (1, 2, 3), vorzugsweise jeder Dummyabschnitt (1, 2, 3) einen Befestigungsbereich aufweist, auf den wenigstens ein Formelement, vorzugsweise nebeneinander mehrere Formelemente befestigbar sind, wobei das jeweilige Formelement
a. entsprechend der individuellen Form des zu dem Formelement korrespondierenden Bereiches an der Extremität eines Patienten ausgebildet ist oder
b. aus einem Satz von mehreren größenverschiedenen oder artverschiedenen Formelementen wählbar ist, insbesondere wobei ein Satz mehrere Formelemente mit stufenweise verschiedenen Größen aufweist.

7. Extremitätendummy nach einem der vorherigen Ansprüche 4 bis 6, **dadurch gekennzeichnet, dass** der jeweilige formkorrespondierende Dummyabschnitt (1, 2, 3) oder formkorrespondierendes Formelement ausgebildet ist als
a. generativ, insbesondere durch 3D-Druck, hergestelltes Bauteil in Abhängigkeit von den korrespondierenden Abschnitt an der Extremität eines Patienten repräsentierenden Daten,
b. spanend aus einem Rohling hergestelltes Bauteil in Abhängigkeit von den korrespondierenden Abschnitt an der Extremität eines Patienten repräsentierenden Daten,
c. Bauteil, dass von einer den korrespondierenden Abschnitt an der Extremität eines Patienten entsprechenden Negativform abgeformt ist.

8. Extremitätendummy nach einem der vorherigen Ansprüche, **dadurch gekennzeichnet, dass** verschiedene Dummyabschnitte (1, 2, 3) und/oder verschiedene Bereiche eines Dummyabschnitts (1, 2, 3) oder verschiedene Formelemente
a. verschiedene Verformbarkeit oder Steifigkeit aufweisen, insbesondere durch Ausbildung aus Materialien mit verschiedenem E-Modul oder Ausbildung mit verschiedenen Versteifungsstrukturen, und/oder
b. verschiedene Oberflächeneigenschaften aufweisen, insbesondere durch unterschiedliche Oberflächenbeschichtungen.

9. Extremitätendummy nach einem der vorherigen Ansprüche, **dadurch gekennzeichnet, dass** der den Unterschenkelabschnitt repräsentierende erste Dummyabschnitt (1) an seinem oberen Ende einen Krafteinleitungsanschluß (6) aufweist, über den eine Kraft (K) in axialer und/oder radialer Richtung des Unterschenkelabschnittes in den Dummyabschnitt (1) einleitbar ist, insbesondere welche die Gewichtskraft eines Patienten nachbildet.

10. Extremitätendummy nach Anspruch 9, **dadurch gekennzeichnet, dass** der Krafteinleitungsanschluß (6) ausgebildet ist durch ein Gehäuseelement (6a), das mit dem ersten Dummyabschnitt (1) drehfest verbunden ist, in welchem ein Schaftelement (6b) drehbar gelagert ist, wobei ein Sensor (7) vorgesehen ist, mit dem die Winkelstellung oder relative Verdrehung zwischen Schaftelement (6b) und Gehäuseelement (6a) messbar ist, vorzugsweise wobei das Schaftelement (6b) relativ zum Gehäuseelement (6a) zumindest zeitweise drehfest fixierbar oder zumindest in der Drehung hemmbar ist.

11. Extremitätendummy nach einem der vorherigen Ansprüche, **dadurch gekennzeichnet, dass** sich gegenüberliegende Gelenkelemente (4, 5) eines Dummyabschnittes (2) oder sich gegenüberliegender Krafteinleitungsanschluß (6) und Gelenkelement (4) eines Dummyabschnittes (1) mittels eines Versteifungselementes verbunden sind, welches eine höhere Steifigkeit aufweist, als das Material des Dummyabschnitts (1, 2), welches das Versteifungselement umgibt.

12. Extremitätendummy nach einem der vorherigen Ansprüche, **dadurch gekennzeichnet, dass** das wenigstens eine Gelenkelement (4) ein Stellelement (7c) aufweist, über welches sich eine Referenzstellung, insbesondere eine Neutralstellung der über das Gelenkelement (4) verbundenen Dummyabschnitte (1, 2) einstellbar ist, insbesondere die vom Sensor (7) ausgebbar ist.

13. Verfahren zum Testen der Funktion einer Orthese (8), die an eine Extremität eines Patienten anlegbar ist, insbesondere zur Ruhigstellung der Extremität, **dadurch gekennzeichnet, dass**
a. die zu testende Orthese (8) an einen Extremitätendummy (1, 2, 3, 4, 5) nach einem der vorherigen Ansprüche angelegt wird, der die Extremität des Patienten, an welche die Orthese (8) anlegebar ist, repräsentiert, und
b. auf den ersten Dummyabschnitt (1) des Extremitätendummys (1, 2, 3, 4, 5) mit dem Krafteinleitungsanschluß (6) eine tordierende Kraft (K, T) ausgeübt wird, um diesen Dummyabschnitt (1, 2, 3) gegenüber wenigstens einen anderen Dummyabschnitt (1, 2, 3), mit dem über ein Gelenkelement (4, 5) eine gelenkige Verbindung besteht, gegen die Wirkung der Orthese (8) zu verlagern, und
c. mit wenigstens einem Sensor (7), der wenigstens einer Gelenkachse (G1, G2, G3) des Gelenkelementes (4, 5) zugeordnet ist, die Lage oder Lageänderung der Dummyabschnitte (1, 2, 3) zueinander gemessen wird, insbesondere in Abhängigkeit der ausgeübten Kraft (K, T) und/oder eines Lage-Parameters, vorzugsweise eine Winkelstellung, des die Kraft (K, T) ausübenden Aktors.

14. Verfahren nach Anspruch 13, **dadurch gekennzeichnet, dass** der Extremitätendummy (1, 2, 3, 4, 5) vor oder nach dem Anlegen der Orthese (8) in eine Testvorrichtung (10) eingesetzt wird, wobei die Testvorrichtung (10) wenigstens ein mit einem Aktor betätigbares Betätigungselement (9) aufweist, mit dem auf wenigstens einen der Dummyabschnitte (1, 2, 3) eine Kraft (K, T) ausgeübt wird.

15. Verfahren nach Anspruch 14, **dadurch gekennzeichnet, dass** der Extremitätendummy (1, 2, 3, 4, 5) eine Unterschenkelextremität repräsentiert und die Testvorrichtung (10) ein Trittflächenelement (9) aufweist, auf welches der wenigstens eine Dummyabschnitt (2, 3) mit der angelegten Orthese (8) aufgestellt wird, der den Mittelfußbereich und/oder Zehenbereich repräsentiert und die Testvorrichtung (10) ein Halteelement (11) aufweist, mit dem eine haltende, tordierende oder eine Gewichtskraft simulierende Kraft (T, K) auf das obere Ende des Dummyabschnitts (1) ausgeübt wird, der den Unterschenkelabschnitt repräsentiert, wobei
a. das Trittflächenelement (9) um eine zur Trittfläche parallele Achse (9a) verschwenkt wird, insbesondere um ein Fußabrollen beim Gehen zu simulieren, oder
b. das Trittflächenelement (9) vor der Krafteinleitung auf das obere Ende des Dummyabschnitts (1), der den Unterschenkelabschnitt repräsentiert, in einer eine vorbestimmte Phase der Fußabrollung beim Gehen entsprechenden Winkel-Lage fixiert wird, und anschließend das obere Ende mit einer Kraft (K) belastet wird, oder
c. die Kraft (K) mittelbar über ein Gelenkelement (14) auf das obere Ende des Dummyabschnittes (1) ausgeübt wird, insbesondere den Schaft (6b) des Krafteinleitungselementes (6), während die Gelenkstellung des Gelenkelements (14) stufenweise oder kontinuierlich verstellt wird oder die Gelenkstellung vor der Krafteinleitung auf das obere Ende des Dummyabschnitts (1), der den Unterschenkelabschnitt repräsentiert, in einer eine vorbestimmte Phase der Fußabrollung beim Gehen entsprechenden Winkel-Lage fixiert wird, und anschließend das obere Ende mit einer Kraft (K) belastet wird, oder
d. der den Mittelfußbereich und/oder Zehenbereich repräsentierende Dummyabschnitt (2, 3) auf dem Trittflächenelement (9, 13) drehfest um die Längsachse des Unterschenkelabschnittes (1) fixiert wird und auf den Dummyabschnitt (1), der den Unterschenkelabschnitt repräsentiert eine diesen um dessen Längsachse tordierende Kraft (T) ausgeübt wird,
wobei die Winkelstellung und/oder Winkeländerung wenigstens eines Dummyabschnittes (1, 2, 3) gegenüber einem anderen, mit dem wenigstens einen Sensor (7) in dem diese Dummyabschnitte (1, 2, 3) verbindenden Gelenkelement (4, 5) gemessen wird, insbesondere in Abhängigkeit der aufgewendeten Kraft (K, T).

16. Verfahren nach einem der vorherigen Ansprüche 13 bis 15, **dadurch gekennzeichnet, dass** der Extremitätendummy (1, 2, 3, 4, 5) zumindest bereichsweise an die Extremität eines Patienten individuell angepasst ist/wird, so dass der Extremitätendummy (1, 2, 3, 4, 5) in den angepassten Bereichen der Form der Extremität des Patienten entspricht, insbesondere wobei auch die zu testende Orthese (8) zumindest bereichsweise an die Extremität eines Patienten individuell angepasst ist/wird, insbesondere durch zumindest bereichsweise Ausbildung der Orthese (8) als Negativform, die zur Form der Extremität des Patienten korrespondiert, und die Orthese (8), insbesondere die angepasste Orthese (8) an den angepassten Extremitätendummy (1, 2, 3, 4, 5) angelegt wird.

17. Verfahren nach Anspruch 16, **dadurch gekennzeichnet, dass** der Extremitätendummy (1, 2, 3, 4, 5) nur in denjenigen Bereichen an die Extremität eines Patienten individuell angepasst ist/wird, in denen auch die Orthese (8) angepasst ist/wird.

## Claims

1. Extremity dummy which forms a lower-leg dummy (1, 2, 3, 4, 5), comprising a first (1) and a second (2) dummy section (1, 2), which are connected in an articulated manner by a first joint element (4) that has at least one axis of rotation (G1, G2) and that represents the ankle joint, wherein
a. the first dummy section (1) represents a lower-leg section (1) of a lower-leg extremity, and
b. the second dummy section (2) represents a foot section, in particular a metatarsal section (2) and/or rear foot section, of a lower-leg extremity,
**characterized in that** the first joint element represents the upper and lower ankle joint, and the mobility in the physiological human joint can be simulated by coupling of at least two joint axes in the first joint element, wherein each of the plurality of axes of rotation (G1, G2) of the joint element (4) is assigned a sensor (7) by which the angular position or change in angle of the connected dummy sections (1, 2) with respect to the assigned axis of rotation (G1, G2) can be measured, and the first dummy section (1) representing the lower-leg section has, at its upper end, a force introduction connection (6) via which a force (T) can be introduced into the dummy section (1) and by which the dummy section (1) can be twisted about its axial extent.

2. Extremity dummy according to Claim 1, **characterized in that** a third dummy section (3) is provided, which is connected in an articulated manner to the second dummy section (2) by a second joint element (5), preferably a joint element (5) having only one axis of rotation (G3), wherein the at least one axis of rotation (G3), preferably the precisely one axis of rotation (G3), of the second joint element (5) is assigned a sensor (7) for measuring the angular position or change in angle of the dummy sections (2, 3), connected to the second joint element (5), with respect to the assigned axis of rotation (G3).

3. Extremity dummy according to Claim 2, **characterized in that** the third dummy section (3) represents a toe section of a lower-leg extremity, and the second joint element (5) represents the row of metatarsal joints.

4. Extremity dummy according to one of the preceding claims, **characterized in that** at least one of a plurality of dummy sections (1, 2, 3), preferably each dummy section (1, 2, 3), is designed, at least in some regions, particularly at least in those regions contactable by an orthosis (8), preferably completely, in a manner preferably corresponding to the individual shape of the section corresponding to the dummy section (1, 2, 3) or of the region on the extremity of a patient corresponding to the region of the dummy section (1, 2, 3).

5. Extremity dummy according to Claim 4, **characterized in that** the extremity dummy (1, 2, 3, 4, 5) is assembled from a plurality of dummy sections (1, 2, 3), wherein each dummy section (1, 2, 3) to be used can be selected from a set of dummy sections (1, 2, 3), in particular which all represent the same extremity section, and dummy sections (1, 2, 3) of different sizes and/or types are present in each set.

6. Extremity dummy according to Claim 4, **characterized in that** at least one of the dummy sections (1, 2, 3), preferably each dummy section (1, 2, 3), has an attachment region to which can be attached at least one shape element, preferably a plurality of shape elements next to one another, wherein the respective shape element
a. is designed corresponding to the individual shape of the region, on the extremity of a patient, corresponding to the shape element, or
b. can be selected from a set of several shape elements of different sizes or different types, in particular wherein a set has a plurality of shape elements of incrementally different sizes.

7. Extremity dummy according to one of Claims 4 to 6, **characterized in that** the respective shape-corresponding dummy section (1, 2, 3) or shape-corresponding shape element is designed as
a. a component produced additively, in particular by 3D printing, as a function of data representing the corresponding section on the extremity of a patient,
b. a component produced from a blank by machining, as a function of data representing the corresponding section on the extremity of a patient, or
c. a component moulded from a negative mould corresponding to the corresponding section on the extremity of a patient.

8. Extremity dummy according to one of the preceding claims, **characterized in that** different dummy sections (1, 2, 3) and/or different regions of a dummy section (1, 2, 3) or different shape elements
a. have different deformability or stiffness, in particular by being made from materials with different moduli of elasticity or being configured with different reinforcement structures, and/or
b. have different surface properties, in particular through different surface coatings.

9. Extremity dummy according to one of the preceding claims, **characterized in that** the first dummy section (1), representing the lower-leg section, has at its upper end a force introduction connection (6) via which a force (K) in the axial and/or radial direction of the lower-leg section can be introduced into the dummy section (1), in particular which force simulates the weight of a patient.

10. Extremity dummy according to Claim 9, **characterized in that** the force introduction connection (6) is formed by a housing element (6a) which is connected for conjoint rotation to the first dummy section (1) and in which a shaft element (6b) is rotatably mounted, wherein a sensor (7) is provided by which the angular position or relative rotation between shaft element (6b) and housing element (6a) can be measured, preferably wherein the shaft element (6b) can be at least temporarily fixed non-rotatably relative to the housing element (6a) or can at least be inhibited in terms of rotation.

11. Extremity dummy according to one of the preceding claims, **characterized in that** mutually opposite joint elements (4, 5) of a dummy section (2), or a mutually opposite force introduction connection (6) and joint element (4) of a dummy section (1), are connected by a reinforcement element which has a greater rigidity than the material of the dummy section (1, 2) surrounding the reinforcement element.

12. Extremity dummy according to one of the preceding claims, **characterized in that** the at least one joint element (4) has an adjustment element (7c) via which a reference position, in particular a neutral position, of the dummy sections (1, 2) connected via the joint element (4) can be adjusted and in particular can be output via the sensor (7).

13. Method for testing the function of an orthosis (8) which can be applied to an extremity of a patient, in particular for immobilizing the extremity, **characterized in that**
a. the orthosis (8) to be tested is applied to an extremity dummy (1, 2, 3, 4, 5) according to one of the preceding claims, which dummy represents the extremity of the patient to which the orthosis (8) can be applied, and
b. a twisting force (K, T) is exerted on the first dummy section (1) of the extremity dummy (1, 2, 3, 4, 5) by the force introduction connection (6), so as to move this dummy section (1, 2, 3), counter to the action of the orthosis (8), with respect to at least one other dummy section (1, 2, 3) to which there is an articulated connection via a joint element (4, 5), and
c. the position or change in position of the dummy sections (1, 2, 3) with respect to one another is measured with at least one sensor (7) which is assigned to at least one joint axis (G1, G2, G3) of the joint element (4, 5), in particular as a function of the exerted force (K, T) and/or of a position parameter, preferably an angular position, of the actuator exerting the force (K, T).

14. Method according to Claim 13, **characterized in that,** before or after the application of the orthosis (8), the extremity dummy (1, 2, 3, 4, 5) is inserted into a testing device (10), the testing device (10) having at least one actuation element (9) which can be actuated by an actuator and by which a force (K, T) is exerted on at least one of the dummy sections (1, 2, 3).

15. Method according to Claim 14, **characterized in that** the extremity dummy (1, 2, 3, 4, 5) represents a lower-leg extremity, and the testing device (10) has a tread element (9) on which the at least one dummy section (2, 3) is placed with the applied orthosis and which represents the metatarsal region and/or toe region, and the testing device (10) has a retaining element (11) configured to exert a retaining, twisting or weight-simulating force (T, K) on the upper end of the dummy section (1), which represents the lower-leg section, wherein
a. the tread element (9) is pivoted about an axis (9a) parallel to the tread, in particular in order to simulate a heel-to-toe roll when walking, or
b. the tread element (9), prior to force being introduced at the upper end of the dummy section (1) that represents the lower-leg section, is fixed in an angular position corresponding to a predetermined phase of the heel-to-toe roll when walking, and then the upper end is loaded with a force (K), or
c. the force (K) is exerted indirectly, via a joint element (14), on the upper end of the dummy section (1), in particular on the shaft (6b) of the force introduction element (6), while the joint position of the joint element (14) is adjusted incrementally or continuously, or the joint position, prior to the force introduction on the upper end of the dummy section (1) that represents the lower-leg section, is fixed in an angular position corresponding to a predetermined phase of the heel-to-toe roll when walking, and then the upper end is loaded with a force (K), or
d. the dummy section (2, 3) representing the metatarsal region and/or toe region is fixed in rotation on the tread element (9) about the longitudinal axis of the lower-leg section (1), and a force (T) twisting the dummy section (1) about the longitudinal axis thereof is exerted on the dummy section (1) representing the lower-leg section,
wherein the angular position and/or change in angle of at least one dummy section (1, 2, 3) with respect to another is measured, in particular as a function of the applied force (K, T), by the at least one sensor (7) in the joint element (4, 5) connecting these dummy sections (1, 2, 3).

16. Method according to one of Claims 13 to 15, **characterized in that** the extremity dummy (1, 2, 3, 4, 5) is individually adapted, at least in some regions, to the extremity of a patient, such that the extremity dummy (1, 2, 3, 4, 5), in the adapted regions, corresponds to the shape of the extremity of the patient, in particular wherein the orthosis (8) to be tested is also individually adapted, at least in some regions, to the extremity of a patient, in particular by the orthosis (8) being configured, at least in some regions, as a negative mould that corresponds to the shape of the extremity of the patient, and the orthosis (8), in particular the adapted orthosis (8), is applied to the adapted extremity dummy (1, 2, 3, 4, 5).

17. Method according to Claim 16, **characterized in that** the extremity dummy (1, 2, 3, 4, 5) is individually adapted to the extremity of a patient only in those regions in which the orthosis (8) is also adapted.

## Revendications

1. Extrémité artificielle, qui forme un bas de jambe artificiel (1, 2, 3, 4, 5), ladite extrémité artificielle comprenant des première (1) et deuxième (2) portions artificielles (1, 2), qui sont reliées de manière articulée au moyen d'un premier élément d'articulation (4) qui comporte au moins un axe de rotation (G1, G2) et qui représente l'articulation de la cheville,
a. la première portion artificielle (1) représentant une portion formant bas de jambe (1) d'une extrémité de bas de jambe, et
b. la deuxième portion artificielle (2) représentant une portion formant pied, en particulier une portion formant pied médian (2) et/ou une portion formant pied arrière d'une extrémité de bas de jambe,
**caractérisée en ce que** le premier élément d'articulation représente l'articulation supérieure et l'articulation inférieure de la cheville et la mobilité dans l'articulation physiologique de la personne peut être simulée par accouplement d'au moins deux axes d'articulation dans le premier élément d'articulation, chacun des plusieurs axes de rotation (G1, G2) de l'élément d'articulation (4) étant associé à un capteur (7) qui permet de mesurer la position angulaire ou la variation angulaire des portions artificielles reliées (1, 2) par rapport à l'axe de rotation associé (G1, G2) et la première portion artificielle (1), qui représente la portion formant bas de jambe, comportant à son extrémité supérieure une liaison d'introduction de force (6) , par laquelle une force (T) peut être introduite dans la portion artificielle (1) avec laquelle la portion artificielle (1) peut être tordue autour de son extension axiale.

2. Extrémité artificielle selon la revendication 1, **caractérisée en ce qu'**une troisième portion artificielle (3) est prévue qui est reliée de manière articulée à la deuxième portion artificielle (2) au moyen d'un deuxième élément d'articulation (5), de préférence d'un élément d'articulation (5) comportant un seul axe de rotation (G3), l'au moins un, de préférence exactement un, axe de rotation (G3) du deuxième élément d'articulation (5) étant associé à un capteur (7) qui permet de mesurer la position angulaire ou la variation angulaire des portions artificielles (2, 3) reliées au deuxième élément d'articulation (5) par rapport à l'axe de rotation associé (G3).

3. Extrémité artificielle selon la revendication 2, **caractérisée en ce que** la troisième portion artificielle (3) représente une portion formant orteil d'une extrémité de bas de jambe et le deuxième élément d'articulation (5) représentant la rangée d'articulation de pied médian.

4. Extrémité artificielle selon l'une des revendications précédentes, **caractérisée en ce qu'**au moins une des plusieurs portions artificielles (1, 2, 3), de préférence chaque portion artificielle (1, 2, 3), est conçue au moins par zones, en particulier au moins dans les zones qui peuvent venir en contact, de préférence entièrement, avec une orthèse (8), en fonction de la forme individuelle, de préférence conformément à celle-ci, de la portion correspondant à la portion artificielle (1, 2, 3) ou de la zone située à l'extrémité d'un patient et correspondant à la zone de la portion artificielle (1, 2, 3).

5. Extrémité artificielle selon la revendication 4, **caractérisée en ce que** l'extrémité artificielle (1, 2, 3, 4, 5) comprend plusieurs portions artificielles (1, 2, 3), chaque portion artificielle (1, 2, 3) à utiliser pouvant être sélectionnée parmi un ensemble de portions artificielles (1, 2, 3), en particulier qui représentent toutes la même portion d'extrémité et chaque ensemble comprenant des portions artificielles (1, 2, 3) de différentes tailles et/ou différents types.

6. Extrémité artificielle selon la revendication 4, **caractérisée en ce qu'**au moins une des portions artificielles (1, 2, 3), de préférence chaque portion artificielle (1, 2, 3), comporte une zone de fixation sur laquelle au moins un élément de forme peut être fixé, de préférence plusieurs éléments de forme peuvent être fixés côte à côte, l'élément de forme respectif
a. étant conçu conformément à la forme individuelle de la zone située à l'extrémité d'un patient et correspondant à l'élément de forme ou
b. pouvant être choisi parmi un ensemble de plusieurs éléments de forme de tailles différentes ou de types différents, notamment un ensemble comportant plusieurs éléments de forme de tailles progressivement différentes.

7. Extrémité artificielle selon l'une des revendications précédentes 4 à 6, **caractérisée en ce que** la portion artificielle de forme correspondante (1, 2, 3) ou l'élément de forme correspondant est conçu(e) comme
a. un composant réalisé de manière générative, notamment par impression 3D, en fonction de données représentant la portion correspondante située sur l'extrémité d'un patient,
b. un composant usiné à partir d'une ébauche en fonction de données représentant la portion correspondante située à l'extrémité d'un patient,
c. un composant qui est moulé à partir d'un moule négatif correspondant à la portion correspondante située à l'extrémité d'un patient.

8. Extrémité artificielle selon l'une des revendications précédentes, **caractérisée en ce que** différentes portions artificielles (1, 2, 3) et/ou différentes zones d'une portion artificielle (1, 2, 3) ou différents éléments de forme
a. présentent différentes déformabilités ou raideurs, notamment par formation à partir de matières ayant différents modules d'élasticité ou par formation avec différentes structures de rigidification, et/ou
b. présentent différentes propriétés de surface, notamment en raison de différents revêtements de surface.

9. Extrémité artificielle selon l'une des revendications précédentes, **caractérisée en ce que** la première portion artificielle (1) représentant la portion formant bas de jambe comporte à son extrémité supérieure une liaison d'introduction de force (6) par laquelle une force (K), en particulier qui simule le poids d'un patient, peut être introduite dans la portion artificielle (1) dans une direction axiale et/ou radiale de la partie formant bas de jambe.

10. Extrémité artificielle selon la revendication 9, **caractérisée en ce que** la liaison d'introduction de force (6) est formée par un élément de boîtier (6a) qui est relié solidairement en rotation à la première portion artificielle (1) et dans lequel un élément formant tige (6b) est monté de manière rotative, un capteur (7) étant prévu qui permet de mesurer la position angulaire ou la rotation relative entre l'élément formant tige (6b) et l'élément de boîtier (6a), de préférence l'élément formant tige (6b) pouvant être fixé solidairement en rotation au moins temporairement par rapport à l'élément de boîtier (6a) ou pouvant au moins être bloqué en rotation.

11. Extrémité artificielle selon l'une des revendications précédentes, **caractérisée en ce que** des éléments d'articulation opposés (4, 5) d'une portion artificielle (2) ou une liaison d'introduction de force opposée (6) et un élément d'articulation (4) d'une portion artificielle (1) sont reliés au moyen d'un élément de rigidification qui présente une rigidité supérieure à celle de la matière de la portion artificielle (1, 2) qui entoure l'élément de rigidification.

12. Extrémité artificielle selon l'une des revendications précédentes, **caractérisée en ce que** l'au moins un élément d'articulation (4) comporte un élément de réglage (7c) qui permet de régler une position de référence, en particulier une position neutre, des portions artificielles (1, 2) reliées par le biais de l'élément d'articulation (4), en particulier qui peut être fournie par le capteur (7).

13. Procédé de test du fonctionnement d'une orthèse (8), qui peur être placée à une extrémité d'un patient, notamment pour immobiliser l'extrémité, **caractérisé en ce que**
a. l'orthèse (8) à tester est placée à une extrémité artificielle (1, 2, 3, 4, 5) selon l'une des revendications précédentes, qui représente l'extrémité du patient sur laquelle l'orthèse (8) peut être placée, et
b. une force de torsion (K, T) est exercée sur la première portion artificielle (1) de l'extrémité artificielle (1, 2, 3, 4, 5) avec la liaison d'introduction de force (6) afin de déplacer cette portion artificielle (1, 2, 3) par rapport à au moins une autre portion artificielle (1, 2, 3), avec laquelle une liaison articulée est formée par le biais d'un élément d'articulation (4, 5), contre l'action de l'orthèse (8), et
c. au moins un capteur (7) est prévu qui est associé à au moins un axe d'articulation (G1, G2, G3) de l'élément d'articulation (4, 5) et qui mesure la position ou la variation de position des portions artificielles (1, 2, 3) l'une par rapport à l'autre, notamment en fonction de la force exercée (K, T) et/ou d'un paramètre de position, de préférence une position angulaire, de l'actionneur exerçant la force (K, T).

14. Procédé selon la revendication 13, **caractérisé en ce que** l'extrémité artificielle (1, 2, 3, 4, 5) est insérée dans un dispositif de test (10) avant ou après la mise en place de l'orthèse (8), le dispositif de test (10) comportant au moins un élément d'actionnement (9) qui peut être actionné par un actionneur et qui permet d'exercer une force (K, T) sur l'une au moins des portions artificielles (1, 2, 3).

15. Procédé selon la revendication 14, **caractérisé en ce que** l'extrémité artificielle (1, 2, 3, 4, 5) représente une extrémité de bas de jambe et le dispositif de test (10) comporte un élément formant surface de marche (9) sur lequel est placée l'au moins une portion artificielle (2, 3), pourvue de l'orthèse (8), qui représente la zone de pied médian et/ou la zone d'orteils et le dispositif de test (10) comporte un élément de retenue (11) qui permet d'exercer une force de retenue, de torsion ou de simulation de poids (T, K) sur l'extrémité supérieure de la portion artificielle (1), qui représente la portion de bas de jambe,
a. l'élément formant surface de marche (9) pivotant sur un axe (9a) parallèle à la surface de marche, en particulier pour simuler le déroulement du pied lors de la marche, ou
b. avant l'introduction de la force, l'élément formant surface de marche (9) est fixé à l'extrémité supérieure de la portion artificielle (1), qui représente la portion formant base de jambe, dans une position angulaire correspondant à une phase prédéterminée du déroulement du pied lors de la marche, puis l'extrémité supérieure est soumise à une force (K), ou
c. la force (K) étant exercée indirectement par le biais d'un élément d'articulation (14) sur l'extrémité supérieure de la portion artificielle (1), en particulier la tige (6b) de l'élément d'introduction de force (6), tandis que la position d'articulation de l'élément d'articulation (14) est ajustée progressivement ou en continu ou la position d'articulation est fixée avant l'introduction de force à l'extrémité supérieure de la portion artificielle (1), qui représente la portion formant bas de jambe, dans une position angulaire correspondant à une phase prédéterminée du déroulement du pied lors de la marche, puis l'extrémité supérieure étant soumise à une force (K), ou
d. la portion artificielle (2, 3), qui représente la zone de pied médian et/ou la zone d'orteils, étant fixée sur l'élément formant surface de marche (9, 13) solidairement en rotation sur l'axe longitudinal de la portion formant bas de jambe (1) et une force étant exercée sur la portion artificielle (1), qui représente le bas de jambe, laquelle force (T) génère une torsion sur l'axe longitudinal de ladite portion artificielle, la position angulaire et/ou la variation angulaire d'au moins une portion artificielle (1, 2, 3) par rapport à une autre étant mesurée, notamment en fonction de la force appliquée (K, T), à l'aide d'au moins un capteur (7) situé dans l'élément l'articulation (4, 5) qui relie ces portions artificielles (1, 2, 3).

16. Procédé selon l'une des revendications précédentes 13 à 15, **caractérisé en ce que** l'extrémité artificielle (1, 2, 3, 4, 5) est adaptée individuellement à l'extrémité d'un patient au moins par zones de sorte que l'extrémité artificielle (1, 2, 3, 4, 5) corresponde dans les zones adaptées à la forme de l'extrémité du patient, en particulier l'orthèse (8) à tester étant également adaptée individuellement à l'extrémité d'un patient au moins par zones, notamment par conformation de l'orthèse (8) au moins par zones en forme négative qui correspond à la forme de l'extrémité du patient, et l'orthèse (8), en particulier l'orthèse adaptée (8), étant placée sur l'extrémité artificielle adaptée (1, 2, 3, 4, 5).

17. Procédé selon la revendication 16, **caractérisé en ce que** l'extrémité artificielle (1, 2, 3, 4, 5) est adaptée individuellement à l'extrémité d'un patient uniquement dans les zones dans lesquelles l'orthèse (8) est également adaptée.
